(19) Europäisches Patentamt
European Patent Office
Office européen des brevets

(11) Veröffentlichungsnummer: **0 258 617 B1**

# (12) EUROPÄISCHE PATENTSCHRIFT

(45) Veröffentlichungstag der Patentschrift: **18.03.92**

(51) Int. Cl.5: **C07D 211/88**, C07D 401/04, A61K 31/44, A61K 31/445

(21) Anmeldenummer: **87110814.8**

(22) Anmeldetag: **25.07.87**

Die Akte enthält technische Angaben, die nach dem Eingang der Anmeldung eingereicht wurden und die nicht in dieser Patentschrift enthalten sind.

## (54) 3,3-Disubstituierte-piperidin-2,6-dion-Derivate.

(30) Priorität: **31.07.86 DE 3625415**

(43) Veröffentlichungstag der Anmeldung:
**09.03.88 Patentblatt 88/10**

(45) Bekanntmachung des Hinweises auf die Patenterteilung:
**18.03.92 Patentblatt 92/12**

(84) Benannte Vertragsstaaten:
**AT BE CH DE ES FR GB GR IT LI LU NL SE**

(56) Entgegenhaltungen:
**EP-A- 0 169 062**
**AT-B- 174 909**
**DD-A- 16 295**
**DE-A- 1 445 652**

(73) Patentinhaber: **MADAUS Aktiengesellschaft**
**Ostmerheimer Strasse 198 Postfach 91 05 55**
**W-5000 Köln 91(DE)**

(72) Erfinder: **Hartmann, Rolf Wolfgang, Dr.**
**Am Mühlberg 3**
**W-8403 Bad Abbach(DE)**
Erfinder: **Batzl, Christine**
**Bäckerstrasse 7a**
**d-8460 Schwandorf(DE)**

EP 0 258 617 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Hemmstoffe der Östrogenbiosynthese stellen ein neues Therapiekonzept zur Behandlung östrogenabhängiger Erkrankungen, insbesondere von östrogenabhängigen Tumoren (zum Beispiel: Mammacarcinom) dar. Durch Hemmstoffe der Aromatase, also des Enzyms, das den letzten Syntheseschritt katalysiert, ist eine selektive Hemmung der Östrogenbiosynthese möglich.

Aminoglutethimid (3- [4-Aminophenyl]-3-ethyl-piperidin-2,6-dion) ist der bislang einzige therapeutisch eingesetzte Hemmstoff der Aromatase.

Aufgabe der Erfindung ist daher die Bereitstellung von verbesserten Aromataseeinhibitoren zur Behandlung von östrogenabhängigen Erkrankungen, insbesondere von hormonabhängigen Tumoren.
Die erfindungsgemäßen Verbindungen stellen solche verbesserten Aromataseeinhibitoren dar. Sie besitzen eine gleiche beziehungsweise ähnliche Wirkung wie die bekannte Verbindung Aminoglutethimid, sind jedoch im Gegensatz hierzu in erheblich niedrigerer Dosierung wirksam, so daß zum Beispiel störende Nebenwirkungen (zum Beispiel Desmolasehemmung) der bekannten Verbindung nicht oder kaum noch auftreten. Außerdem treten bei den erfindungsgemäßen Verbindungen keine unerwünschten sedativen Wirkungen auf.

Die erfindungsgemäßen Verbindungen sind daher zum Beispiel zur Behandlung von östrogenabhängigen Tumoren wie Mammacarcinom, Endometriumcarcinom, aber auch von anderen östrogenabhängigen Erkrankungen wie Endometriose und idiopathische Oligospermie geeignet. Weiterhin stellen sie potentielle Ovulationshemmer und Geburtsauslöser dar und sind zur Behandlung und Vorbeugung der benignen Prostatahyperplasie geeignet.

Als $C_3$-$C_{10}$-Cycloalkylrest kommt insbesondere ein $C_4$-$C_8$-Cycloalkylrest in Frage, zum Beispiel der Cyclobutyl-, Cyclopentyl-, Cyclohexyl-, Cycloheptyl- und Cyclooctylrest.
Falls der $C_3$-$C_{10}$-Cycloalkylrest ungesättigt ist, enthält er vorzugsweise eine Doppelbindung, wobei es sich hierbei insbesondere um Ringe mit 5 - 10 Ringgliedern vorzugsweise 6 - 8 Ringgliedern handelt.

Als $C_3$-$C_{12}$-Alkenylrest, der gerade oder verzweigt sein kann, kommt insbesondere jeweils ein $C_3$-$C_8$ Alkenylrest, vorzugsweise ein $C_3$-$C_6$-Alkenylrest in Frage.

Besonders vorteilhaft sind Verbindungen der Formel I, worin A ein 4-Amino-phenylrest und $R_1$ ein $C_3$-$C_{10}$-Cycloalkylrest ist.

Die erfindungsgemäßen Verbindungen enthalten ein gegebenenfalls auch zwei oder auch drei asymmetrische Kohlenstoffatome. Bei der Synthese können daher Gemische von Diastereoisomeren auftreten. Man kann diese Gemische auf üblichem Weg, beispielsweise durch Umkristallisieren, trennen. Die Enantiostereoisomeren lassen sich aus den reinen Racematen mit Hilfe optisch aktiver Hilfssubstanzen (zum Beispiel optisch aktive Säuren) in an sich bekannter Weise aufspalten. Es ist aber auch möglich, bei der Synthese optisch aktive beziehungsweise diastereomere Ausgangsstoffe einzusetzen, wobei die entsprechenden enantiostereomeren beziehungsweise die diastereomeren Endprodukte erhalten werden. Für die praktische pharmazeutische Verwendung kommen reine Isomere oder Isomerengemische in Frage.

Die Formel I umfasst sämtliche möglichen Enantiomere und Diastereomere.

Die Cyclisierung des erfindungsgemäßen Verfahrens wird in einem höher siedenden Lösungsmittel bei Temperaturen zwischen 50 und 200 ° C, insbesondere 80 und 150 ° C, gegebenenfalls in Gegenwart von Kondensationsmitteln durchgeführt. Als Kondensationsmittel kommen in Frage starke Mineralsäuren ($H_2SO_4$, HCl, Polyphosphorsäure) oder starke Basen ($NH_3$). Im Falle der Cyclisierung von Diestern (X und Y bedeuten jeweils $COOC_1$-$C_6$-Alkylgruppe) ist beispielsweise die Verwendung von $NH_3$ oder Harnstoff erforderlich.

Als Lösungsmittel kommen in Frage: niedere aliphatische Carbonsäuren mit 1-6-C-Atomen wie Ameisensäure, Eisessig, Propionsäure, Anhydride von niederen aliphatischen Carbonsäuren mit 2 bis 4 C-Atomen wie Acetanhydrid; basische Lösungsmittel wie Pyridin oder niedere Alkylamide von niederen aliphatischen Carbonsäuren wie Dimethylformamid, Diethylacetamid, sowie Gemische dieser Mittel. Die Reaktionszeit beträgt beispielsweise 2 - 10 Stunden.
Falls X und/oder Y die Estergruppe CO-$OC_1$-$C_6$-Alkyl bedeuten, besteht der Alkylrest vorzugsweise aus 1 bis 4 C-Atomen, vorzugsweise handelt es sich um die Ethoxycarbonylgruppe.

Falls A ein Phenylrest ist, kann in diesem eine Nitrogruppe durch übliche Nitrierung eingeführt werden.
Als Nitrierungsmittel kommen beispielsweise in Frage:
Salpetersäure oder Salpetersäuregemische mit $H_2SO_4$, $H_3PO_4$, Selensäure, $BF_3$;
Gemischte Anhydride der Salpetersäure (zum Beispiel Acetylnitrat, Benzoylnitrat);
Nitroniumverbindungen (Halogenide, Perchlorat, Tetrafluorborat);
Metallnitrate mit und ohne Zugabe von Säuren;
Salpetersäureester mit und ohne Zugabe von Säuren;

Nitroalkane (zum Beispiel Tetranitromethan);
Salpetrige Säure beziehungsweise deren Derivate (zum Beispiel Ester oder Nitrosoniumhydrogensulfat);
Stickstoffoxide.

Die Nitrierung kann mit oder ohne zusätzliche Lösungsmittel bei Temperaturen zwischen -20 und +100 °C erfolgen. Reaktionszeit zum Beispiel 1 - 80 Stunden. Falls Lösungsmittel verwendet werden, kommen zum Beispiel in Frage:
niedere flüssige aliphatische Chlorkohlenwasserstoffe ($CHCl_3$, $CH_2Cl_2$, $CCl_4$), flüssige Alkane (ligroin), niedere aliphatische Alkohole (Ethanol), niedere aliphatische oder cycloaliphatische Ether (Diethylether), niedere aliphatische Ketone (Aceton), Schwefelkohlenstoff, Nitromethan, Acetonitril.

Für die Reduktion der Nitrogruppe kommt insbesondere die katalytische Hydrierung in Betracht. Als Katalysatoren kommen zum Beispiel in Frage:
Raney-Nickel, Edelmetalle wie Palladium und Platin sowie Verbindungen hiervon (Oxide) mit und ohne Träger (Aktivkohle, Bariumsulfat, Calciumsulfat).

Es empfiehlt sich, die Hydrierung der Nitrogruppe bei Temperaturen zwischen 20 und 120 °C und einem Druck von ungefähr 5 - 100 atü in einem Lösungsmittel, beispielsweise Wasser, niederen Alkoholen, niederen aliphatischen Ethern, gesättigten cyclischen Ethern (Dioxan, Tetrahydrofuran), niederen Carbonsäuren mit 1-4-C-Atomen (Eisessig) oder auch flüssigen Alkanen vorzunehmen. Für die anschliessende Isolierung der reduzierten Verbindungen kann es in manchen Fällen von Vorteil sein, wenn zu Beginn dem zu hydrierenden Gemisch Trockenmittel, wie wasserfreies Natrium- oder Magnesiumsulfat zugesetzt werden. Die Reduktion kann aber auch mit nascierendem Wasserstoff, beispielsweise Zink/Salzsäure, Zinn/Salzsäure, Eisen/Salzsäure oder mit Salzen des Schwefelwasserstoffs Ammonsulfid, Natriumsulfid, Natriumdithionit bei etwa 20 bis 100 °C oder mit aktiviertem Aluminium in wasserhaltigem Ether oder mit Zinn(II)-chlorid/Salzsäure durchgeführt werden.

Herstellung von nichtbekannten Ausgangssubstanzen der Formel II

Die Herstellung solcher Ausgangssubstanzen erfolgt grundsätzlich in analoger Weise wie die Herstellung der Ausgangssubstanzen von Beispiel 1.

In das entsprechende Cyanid A-$CH_2$-CN beziehungsweise den entsprechenden Ester A-$CH_2$-$COOC_1$-$C_6$-Alkyl wird bei Temperaturen zwischen 60 - 120 °C in einem inerten Lösungsmittel (Toluol, Benzol) in Gegenwart von basischen Verbindungen (Alkaliamide, Alkalihydride wie $NaNH_2$, NaH; Alkalialkoholate wie Kalium-tert.-butylat; Butyllithium) der Rest $R_1$ durch Alkylierung eingeführt, unter Verwendung der entsprechenden Halogenide (Chloride, Bromide, Jodide), Sulfate, Sulfonate oder Tosylate mit dem Rest $R_1$ als Alkylierungsmittel. Diese Alkylierung kann auch unter Phasentransferbedingungen erfolgen. Die so erhaltene Verbindung A-CH($R_1$)-X (Bedeutungen von X wie angegeben) wird dann beispielsweise an eine Verbindung $CH_2$ = CH-Y (Y ist CN, $COOC_1$-$C_6$-Alkyl oder $CO_2H$) bei Temperaturen zwischen 20 und 120 °C in Gegenwart von Basen (quartäre Ammoniumverbindungen wie Triton B, Alkalialkoholate, Alkaliamide, Alkalihydroxide wie KOH, NaOH) angelagert. Als wasserfreie Lösungsmittel kommen hierbei in Frage: cyclische Ether (Dioxan, Tetrahydrofuran), Benzol, Toluol, tert.-Butylalkohol, Acetonitril. Die Base kann in äquivalenter Menge zu der CH-aciden Komponente verwendet werden. Falls A der Pyridyl-(4)-rest ist, kann die Ausgangssubstanz A-CH($R_1$)-CN beziehungsweise A-CH($R_1$)-$COOC_1$-$C_6$-Alkyl auch wie folgt erhalten werden:
Umsetzung von A-Cl mit $R_1CH_2X$ (X = CN, $COOC_1$-$C_6$-Alkyl) bei Temperaturen zwischen 60 - 120 °C in einem inerten wasserfreien Lösungsmittel (Benzol, Toluol) in Gegenwart von Basen (Alkaliamide, Alkalihydride wie $NaNH_2$, NaH; Alkalialkoholate wie Kaliumtert.-butylat). Siehe hierzu auch J. Am. Chem. Soc. 73, Seite 4925 (1951).
Beispielsweise kann man wie folgt verfahren:
2-Cycloalkyl-2-(4-pyridyl)acetonitril (2-Alkenyl-....;
2-Cycloalkenyl-....)
0.1 Mol Cycloalkylacetonitril (Alkenyl-.... beziehungsweise Cycloalkenyl-....) und 0.1 Mol $NaNH_2$ werden 2 Stunden bei 80°C in 50 ml Toluol erhitzt. Nach dem Abkühlen gibt man tropfenweise 0.1 mol 4-Chlorpyridin in 15 ml Toluol zu und erhitzt 1 Stunde unter Rückfluß. Nach dem Abkühlen schüttelt man mit 1 normaler HCl aus. Nach Neutralisieren wird mit $CH_2Cl_2$ extrahiert und über $Na_2SO_4$ getrocknet. Das Lösungsmittel wird abgezogen und das Rohprodukt chromatographisch gereinigt.

Oder man setzt das $BF_4$-Salz des N-Triphenylmethyl-pyridinium-Kations gemäß J.C.S. Perkin, Transaction I 2476 (1981) mit der Verbindung $R_1CH_2X$ (X = CN, $COOC_1$-$C_6$-Alkyl, COOH) bei Temperaturen zwischen -80°C und +120°C in einem inerten Lösungsmittel (Toluol, Benzol, cyclische Ether wie Dioxan, Tetrahydrofuran, flüssige Alkane) in Gegenwart von Basen um. Als Basen kommen zum Beispiel in Frage:

Alkaliamide, Alkalihydride ($NaNH_2$, Lithiumdiisopropylamid, Lithiumdiisopropylcyclohexylamid, NaH, Alkalialkoholate (Kalim-tert.-butylat).
Beispielsweise kann man wie folgt verfahren:
2-Cycloalkyl-2-(4-pyridyl)acetonitril (2-Alkenyl-....;
2-Cycloalkenyl....)
Zu einer Lösung von 0.1 Mol Lithiumdiisopropylamid in 200 ml trockenem Tetrahydrofuran wird unter $N_2$-Atmosphäre bei -78° 0.1 mol 2-Cycloalkyl(2-Alkenyl-, 2-Cycloalkenyl-)acetonitril über einen Zeitraum von 2 Minuten zugefügt und die Mischung 40 Minuten bei 0° gerührt. Dann wird bei 0° über einen Zeitraum von 2 Minuten 0.1 mol (40.9 g) Triphenylmethylpyridinium-tetrafluorborat portionsweise zugegeben. Sobald sich alles Pyridiniumsalz gelöst hat, wird das Tetrahydrofuran abgezogen und der Rückstand in 40 ml $CHCl_3$ aufgenommen. Der Extrakt wird filtriert und das Filtrat mit 30 ml Tetrachlorkohlenstoff gemischt. Die Mischung läßt man über Nacht rühren. Nach Abzug des Lösungsmittels wird der Rückstand zweimal chromatographiert ($Al_2O_3$, Grad I, neutral; $CCl_4$ : $CHCl_3$ = 1 : 4; Diethylether : $CHCl_3$ = 1 : 9).

Die erfindungsgemäßen Verbindungen zeigen am DMBA-induzierten Mammacarcinom (postmenopause Modell und an der PMSG-vorbehandelten Sprague-Dawley-Ratte eine gute mammatumorhemmende Absenkung des Östrogenspiegels auf Kastrationsniveau.

DMBA = 7,12-Dimethylbenz a -anthracen
PMSG = Gonadotropin aus dem Serum von schwangeren Stuten

Beispielsweise wird bei obengenannter Versuchsmethode bei einer Dosis von 0.3 mg/kg Körpergewicht SD-Ratte (subcutan) eine 50 % Reduktion des Östradiolspiegels PMSG-geprimter Ratten erzielt.
Die niedrigste, bereits wirksame Dosis in dem obenangegebenen Tierversuch ist beispielsweise
ca. 0.05 mg/kg subcutan
ca. 0.1 mg/kg oral
ca. 0.05 mg/kg intravenös

Als allgemeiner Dosisbereich für die Wirkung (Tierversuch wie oben) kommt beispielsweise in Frage:
0.1 - 1.0 mg/kg oral, insbesondere 0.5
0.05 - 0.5 mg/kg intravenös
0.1 - 1.0 mg/kg subcutan, insbesondere 0.5

Die Wirkungsrichtung der erfindungsgemäßen Verbindungen ist mit der Wirkung des bekannten Arzneimittelwirkstoffs Aminoglutethimid vergleichbar, jedoch bestehen hierzu insbesondere folgende Unterschiede: Die erfindungsgemäßen Verbindungen sind in wesentlich niedriger Dosis wirksam; auf Grund der in dieser Dosis fehlenden Desmolasehemmung muß nicht mit Hydrocortison substituiert werden: es treten keine zentralnervensystem-depressiven Effekte auf.

Indikationen für die die erfindungsgemäßen Verbindungen in Betracht kommen können: hormonabhängige Tumore, wie zum Beispiel Mammacarcinom und Endometriumcarcinom, Endometriose, idiopathische Oligospermie, Ovulationshemmer, benigne Prostatahyperplasie, Geburtsauslöser.

Die pharmazeutischen Zubereitungen enthalten im allgemeinen zwischen 5 bis 50, vorzugsweise 10 bis 20 mg der erfindungsgemäßen aktiven Komponente(n). Als Tagesdosis kommen beispielsweise 50 mg Verbindung I in Frage.

Die Verabreichung kann beispielsweise in Form von Tabletten, Kapseln, Pillen, Dragees, Zäpfchen, Salben, Gelees, Cremes, Puder, Stäubepulver, Aerosolen oder in flüssiger Form erfolgen. Als flüssige Anwendungsformen kommen zum Beispiel in Frage: Ölige oder alkoholische beziehungsweise wässrige Lösungen sowie Suspensionen und Emulsionen. Bevorzugte Anwendungsformen sind Tabletten, die zwischen 10 und 20 mg oder Lösungen, die zwischen 0.5 bis 5 Gewichtsprozent an aktiver Substanz enthalten.

Die Einzeldosis der erfindungsgemäßen aktiven Komponenten kann beispielsweise liegen

a) bei oralen Arzneiformen zwischen 5 bis 50 mg vorzugsweise 10 - 20 mg

b) bei parenteralen Arzneiformen (zum Beispiel intravenös, intramuskulär) zwischen 1 bis 5 ml vorzugsweise 2 - 3 ml

- (Die Dosen sind jeweils bezogen auf die freien Base) -

Beispielsweise können 3 mal täglich 1 bis 2 Tabletten mit einem Gehalt von 5 bis 50 mg wirksamer Substanz oder zum Beispiel bei intravenöser Injektion 1 bis 3 mal täglich eine Ampulle von 1 bis 5 ml Inhalt mit 5 bis 50 mg Substanz empfohlen werden. Bei oraler Verabreichung ist die minimale tägliche Dosis beispielsweise 15 mg; die maximale tägliche Dosis bei oraler Verabreichung soll nicht über 300 mg liegen.

Die akute Toxizität der erfindungsgemäßen Verbindungen an der Maus (ausgedrückt durch die LD 50 mg/kg; Methode nach Miller und Tainter: Proc. Soc. Exper. Biol. a. Med. 57 (1944) 261) liegt beispielsweise bei oraler Applikation oberhalb 600 mg/kg.

Beispiele

Beispiel 1

3-(4-Aminophenyl)-3-cyclohexyl-piperidin-2,6-dion

Eine Mischung aus 0.1 mol 2-Cyclohexyl-2-phenylglutarsäuredinitril, 150 ml konzentrierter $H_2SO_4$ und 500 ml Eisessig wird 6 Stunden im Wasserbad erhitzt. Nach dem Abkühlen gießt man auf Eis und schüttelt mit $CH_2Cl_2$ aus. Die organische Phase wird mit gesättigter $Na_2CO_3$-Lösung, dann mit $H_2O$ gewaschen. Das Lösungsmittel wird abgezogen und das 3-Cyclohexyl-3-phenyl-piperidin-2,6-dion aus Essigester/Ligroin umkristallisiert (Ausbeute 75 % der Theorie).
Zu einer Lösung von 3-Cyclohexyl-3-phenyl-piperidin-2,6-dion (0.05 mol) in 40 g konzentrierter $H_2SO_4$ tropft man bei -10 ° C eine Mischung von 5.5 g konzentrierter $H_2SO_4$ und 5.5 g $HNO_3$ 70 %ig.
Das Reaktionsgemisch wird 2 Stunden bei -10 ° C gehalten. Dann wird auf Eis gegossen und mit $CH_2Cl_2$ extrahiert. Die organische Phase wird mit gesättigter $Na_2CO_3$-Lösung, dann mit $H_2O$ gewaschen und über $Na_2SO_4$ getrocknet.
Nach Abzug des Lösungsmittels wird das erhaltene 3-Cyclohexyl-3-(4-nitrophenyl)-piperidin-2,6-dion aus Toluol umkristallisiert(F 155-7 ° C) (Ausbeute 97 % der Theorie).

Eine Lösung von 3-Cycloalkyl-3-(4-nitrophenyl)-piperidin-2,6-dion (0.01 mol) in 100 ml Ethanol wird mit Palladium auf Aktivkohle versetzt (10 %-ig, 0.1 g). Man läßt unter $H_2$-Atmosphäre so lange schütteln, bis kein Wasserstoff mehr aufgenommen wird. Die Suspension wird durch Umkristallisation aus Toluol gereinigt. F 189 - 190 ° C Ausbeute 73 % der Theorie.

Herstellung der Ausganssubstanzen

2-Cyclohexyl-2-phenyl-acetonitril

Zu einer Lösung von Benzylcyanid (58.5 g, 0.5 mol) und Cyclohexylbromid (0.5 mol) in 200 ml trockenem Benzol wird bei 80 ° C Natriumamid (0.5 mol) zugegeben. Das Reaktionsgemisch wird 2 Stunden erhitzt. Nach Abkühlen gibt man 200 ml $H_2O$ zu, trennt die organische Phase ab, wäscht mit $H_2O$ und trocknet über $Na_2SO_4$.Nach Abzug des Lösungsmittels wird aus Diethylether umkristallisiert. Ausbeute 50 % der Theorie.

2-Cyclohexyl-2-phenyl-glutarsäuredinitril

Zu einer Mischung von 0.3 Mol 2-Cyclohexyl-2-phenyl-acetonitril 0.3 mol eines nichtionogenen Alkylarylpolyetheralkohols beziehungsweise Alkylphenylethers des Polyethylenglykols (Triton B) und 300 ml trockenem Dioxan gibt man bei 100 ° C Acrylnitril (15.9 g, 0,3 mol) zu und kocht 40 Stunden unter Rückfluß. Nach Abzug des Lösungsmittels wird der Rückstand mit $CHCl_3$ extrahiert. Die Chloroformphase wird mit $H_2O$ gewaschen und über $Na_2SO_4$ getrocknet. Das Lösungsmittel wird abgezogen und das ölige Rohprodukt durch Säulenchromatographie unter Stickstoff gereinigt. (Kieselgel, $CH_2Cl_2$ Petrolether = 4 : 1). Ausbeute 55 % der Theorie.

Beispiel 2

3-(4-Aminophenyl)-3-cyclopentyl-piperidin-2,6-dion

Die Verbindung wird analog der Herstellungsweise von Beispiel 1 erhalten, in dem als Ausgangsmaterial 0.1 mol 2-Cyclopentyl-2-phenyl-glutarsäuredinitril eingesetzt wird. Ausbeute 73 % der Theorie. F. 138 - 140 ° C.

Beispiele für galenische Zubereitungen

Tabletten 250 mg Wirkstoff

750 g Wirkstoff nach Beispiel 1, 225 g Lactose, 94 g Maisstärke, 3 g Aerosil werden in einem geeigneten Wirbelschichtgranuliergerät gemischt und mit 120 g einer 10 %igen Gelatinelösung zwecks Granulierung besprüht. Das trockene Granulat, 78 g Maisstärke, 30 g Talkum und 6 g Magnesiumstearat werden durch ein Sieb von 0.8 mm Maschenweite passiert und homogenisiert. Diese Masse wird in üblicher Weise auf einer geeigneten Maschine zu Tabletten mit Bruchrille von 400 mg Gewicht und einem

Durchmesser von 11 mm verpreßt.
1 Tablette enthält 250 mg Wirkstoff.

Kapseln 250 mg Wirkstoff

750 g Wirkstoff nach Beispiel 2, 90 g Lactose, 183 g mikrokristalline Cellulose, 3 g Aerosil, 21 g Talkum und 3 g Magnesiumstearat werden durch ein Sieb (0,8 mm Mascheinweite) gegeben und diese Mischung in einem geeigneten Gerät homogenisiert. Diese Masse wird auf einer geeigneten Kapselmaschine zu 350 mg in Hartgelatinekapseln der Größe 1 abgefüllt.
1 Kapsel enthält 250 mg Wirkstoff.

**Patentansprüche**

1. Verbindungen der allgemeinen Formel

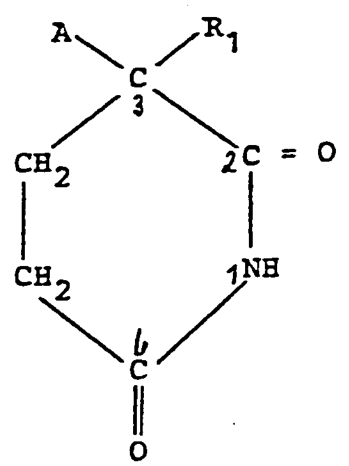

I

worin A ein 4-Amino-phenylrest oder ein Pyridyl-(4)-rest und $R_1$ ein gesättigter oder ungesättigter $C_3$-$C_{10}$-Cycloalkylrest oder ein $C_3$-$C_{12}$ Alkenylrest ist, sowie deren physiologisch verträgliche Salze.

2. Verfahren zur Herstellung von Verbindungen der allgemeinen Formel

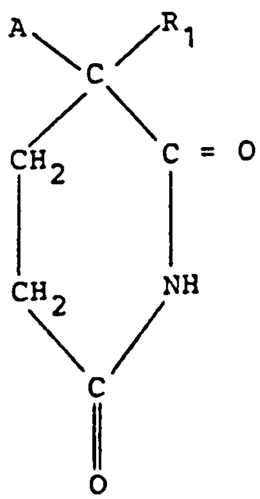

I

worin A ein 4-Amino-phenylrest oder ein Pyridyl-(4)-rest und $R_1$ ein gesättigter oder ein ungesättigter $C_3$-$C_{10}$-Cycloalkylrest oder ein $C_3$-$C_{12}$-Alkenylrest ist, sowie deren physiologisch verträglichen Salzen, dadurch gekennzeichenet,

daß man eine Verbindung der Formel

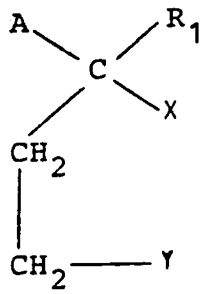

II

worin A und $R_1$ die angegebenen Bedeutungen haben, A außerdem auch ein Phenylrest oder ein 4-Nitrophenylrest sein kann, X und Y gleich oder verschieden sind und CN oder $COOC_1$-$C_6$-Alkyl bedeuten und X oder Y auch COOH sein kann, in Gegenwart von Säure erhitzt, falls A ein Phenylrest ist durch Nitrierung eine Nitrogruppe in die 4-Stellung dieses Phenylrestes einführt und/oder eine Nitrogruppe des 4-Nitrophenylrestes zur Aminogruppe reduziert und die erhaltenen Verbindungen gegebenenfalls in ihre Salze überführt.

**3.** Arzneimittel, enthaltend eine Verbindung der allgemeinen Formel I neben üblichen Träger- und/oder Verdünnungs- beziehungsweise Hilfsstoffen.

**4.** Verfahren zur Herstellung eines Arzneimittels, dadurch gekennzeichnet,
daß eine Verbindung der allgemeinen Formel I mit gebräuchlichen pharmazeutischen Trägerstoffen und/oder Verdünnungsmitteln beziehungsweise sonstigen Hilfsstoffen zu pharmazeutischen Zubereitungen verarbeitet beziehungsweise in eine therapeutisch anwendbare Form gebracht wird.

**5.** Verwendung von Verbindungen der allgemeinen Formel I zur Herstellung von Arzneimitteln.

## Claims

**1.** Compounds of the general formula:

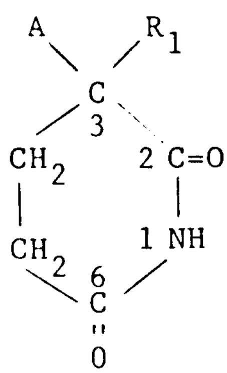

I

wherein A is a 4-aminophenyl radical or a pyridyl-(4) radical and $R_1$ a saturated or unsaturated $C_3$-$C_{10}$-cycloalkyl radical or a $C_3$-$C_{12}$-alkenyl radical, as well as their physiologically compatible salts.

**2.** Process for the preparation of compounds of the general formula

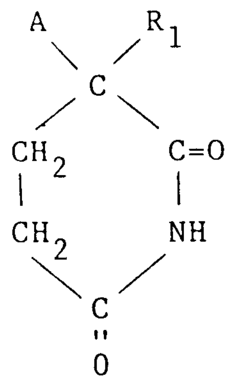

I

wherein A is a 4-aminophenyl radical or a pyridyl-(4) radical and $R_1$ a saturated or unsaturated $C_3$-$C_{10}$-cycloalkyl radical or a $C_3$-$C_{12}$-alkenyl radical, as well as of their physiologically compatible salts, characterised in that one heats a compound of the formula

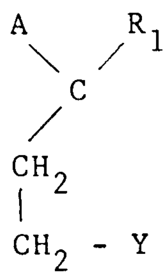

II

wherein A and $R_1$ have the given meanings, A can furthermore also be a phenyl radical or a 4-nitrophenyl radical, X and Y are the same or different and signify CN or $COOC_1$-$C_6$-alkyl and X and Y can also be COOH, in the presence of an acid, if A is a phenyl radical introduces a nitro group into the 4-position of this phenyl radical by nitration and/or reduces a nitro group of the 4-nitrophenyl radical to the amino group and possibly converts the compounds obtained into their salts.

**3.** Medicaments containing compounds of the general formula I, besides usual carrier and/or dilution or adjuvant materials.

**4.** Process for the production of a medicament, characterised in that a compound of the general formula I is worked up to pharmaceutical compositions with conventional pharmaceutical carrier materials and/or dilution agents or other adjuvant materials or is brought into a therapeutically usable form.

**5.** Use of compounds of the general formula I for the production of medicaments.

## Revendications

**1.** Composés de la formule générale

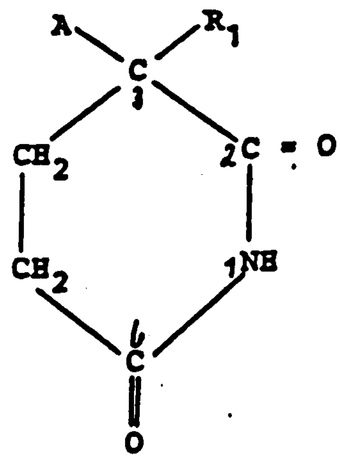

I

dans laquelle A est un radical 4-amino-phényle ou un radical pyridyl-(4) et $R_1$ est un radical cycloalkyle saturé ou insaturé en $C_3$-$C_{10}$ ou un radical alcényle en $C_3$-$C_{12}$ ainsi que leurs sels physiologiquement compatibles.

**2.** Procédé pour la préparation des composés de la formule générale

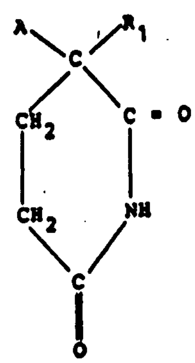

I

dans laquelle A est un radical 4-amino-phényle ou un radical pyridyl-(4) et $R_1$ est un radical cycloalkyle saturé ou insaturé en $C_3$-$C_{10}$ ou un radical alcényle en $C_3$-$C_{12}$, ainsi que leurs sels physiologiquement compatibles,
caractérisé en ce que
l'on fait chauffer un composé de la formule

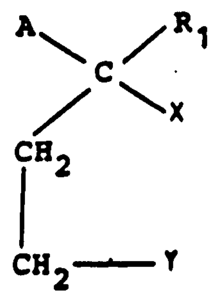

II

dans laquelle A et $R_1$ ont les significations indiquées, A peut en outre être un radical phényle ou un radical 4-nitrophényle, X et Y sont identiques ou différents et CN ou $COOC_1$-$C_6$- signifient alkyle et x ou Y peuvent également être COOH, en présence d'acide, si A est un radical phényle on introduit par

nitration un groupe nitro dans la quatrième position de ce radical phényle et/ou on réduit un groupe nitro du radical 4-nitrophényle en groupe amino et l'on transforme les composés obtenus éventuellement en leurs sels.

**3.** Médicament contenant un composé de la formule générale I en plus des excipients usuels- et/ou des diluants ou adjuvants.

**4.** Procédé pour la préparation d'un médicament, caractérisé en ce que l'on transforme un composé de la formule générale I avec les excipients pharmaceutiques usuels et/ou diluants ou autres adjuvants en préparations pharmaceutiques ou on les met sous une forme thérapeutiquement applicable.

**5.** Utilisation de composés selon la formule générale I pour la préparation de médicaments.